(19) Europäisches Patentamt
European Patent Office
Office européen des brevets

(11) Veröffentlichungsnummer: **0 093 317**
**B1**

# (12) EUROPÄISCHE PATENTSCHRIFT

(45) Veröffentlichungstag der Patentschrift: **12.12.84**

(51) Int. Cl.³: **C 07 C 53/122**, C 07 C 51/41

(21) Anmeldenummer: **83103816.1**

(22) Anmeldetag: **20.04.83**

## (54) Verfahren zur Herstellung von Calciumpropionat.

(30) Priorität: **28.04.82 DE 3215752**

(43) Veröffentlichungstag der Anmeldung: **09.11.83 Patentblatt 83/45**

(45) Bekanntmachung des Hinweises auf die Patenterteilung: **12.12.84 Patentblatt 84/50**

(84) Benannte Vertragsstaaten: **BE DE FR GB NL**

(56) Entgegenhaltungen: **DERWENT JAPANESE PATENTS REPORT, Band 7, Nr. 25, 1968 Seite 1, Teil 5**

(73) Patentinhaber: **BASF Aktiengesellschaft, Carl-Bosch-Strasse 38, D-6700 Ludwigshafen (DE)**

(72) Erfinder: **Merkel, Dieter, Dr., Homburger Strasse 8, D-6700 Ludwigshafen (DE)**
Erfinder: **Muehlthaler, Wolfgang, Dipl.-Ing., Regerstrasse 13, D-6944 Hemsbach (DE)**
Erfinder: **Diem, Hans, Dr., Feldbergstrasse 63, D-6800 Mannheim 25 (DE)**
Erfinder: **Matthias, Guenther, Dr., Meergartenweg 25 a, D-6710 Frankenthal (DE)**

EP 0 093 317 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Calciumpropionat.

Die Herstellung dieser in großem Maßstab zur Konservierung auf dem Lebensmittelsektor und bei der Tierfutterhaltung eingesetzten Verbindung (K. WEISSERMEL u. H. J. ARPE »Industrielle organische Chemie«, S. 115, Verlag Chemie 1976) erfolgt nach den üblichen Synthesemethoden für Carbonsäuresalze, z. B. durch Umsetzung von Carbonaten, Hydroxiden oder Oxiden mit konzentrierten oder verdünnten Carbonsäuren.

Für die Reaktion von Calciumhydroxid mit Propionsäure, die exotherm abläuft, gilt folgende Gleichung:

$$2\,C_2H_5COOH + Ca(OH)_2 \rightarrow Ca(C_2H_5COO)_2 + 2\,H_2O$$

Die Abführung der Reaktionswärme kann teils durch Wärmeübertragung auf zusätzliche Hilfsstoffe, teils durch Verdampfung von Wasser geschehen.

Nach dem Vorschlag der JP-PS 14 684/1968 werden Propionsäure und Calciumhydroxid zusammen mit Wasser zu einer Paste verrieben. Durch Zusatz von trockenem Calciumpropionat erhält man eine teigartige Substanz, die getrocknet wird.

Eine andere Möglichkeit besteht in der Umsetzung von Calciumoxid bzw. -hydroxid mit Propionsäure im Beisein eines mehrwertigen Alkohols, z. B. Glykol (GB-PS 684 155). Weiterhin kann auch in Gegenwart fester, inerter Fremdstoffe gearbeitet werden. So wird in der deutschen Patentanmeldung P 53 194 D die Umsetzung von konzentrierter Propionsäure mit Metalloxiden bzw. -carbonaten unter Hinzugabe von Soja- oder Malzmehl vorgeschlagen.

Auch anorganische Inertstoffe (z. B. Kieselgur oder Talkum) können in feinverteilter Form, unter starker Durchmischung mit den Reaktionspartnern verwendet werden. Um die Reaktionsgeschwindigkeit zu erhöhen, arbeitet man hierbei im warmen Luftstrom, gegebenenfalls im Fließbett oder in der Wirbelschicht (DE-PS 1 217 381).

Die Verwendung von inerten Hilfsstoffen scheidet aber dort aus, wo, wie im vorliegenden Fall, reines Calciumpropionat hergestellt werden soll. Hier ist es dann zweckmäßig, die Reaktionswärme gezielt zur Verdampfung des entstehenden Wassers auszunützen.

Um die bei der Bildung von 1 kg Calciumpropionat gleichzeitig entstehende Menge von 0,194 kg Wasser zu verdampfen, benötigt man 439 kJ. Dazu ist die pro kg Calciumpropionat freiwerdende Wärmemenge von 670 kJ ausreichend.

Da sich aber bei kontinuierlicher Arbeitsweise im Reaktor alle Reaktionskomponenten (Propionsäure, Calciumhydroxid, Calciumpropionat und Wasser) immer gleichzeitig befinden, verdampft dabei nicht reines Wasser, sondern ein azeotropes Gemisch, das ca. 75 Gew.-% Wasser und ca. 25 Gew.-% Propionsäure enthält. Es stellt sich somit das Problem, dieses kontinuierlich anfallende Propionsäure-Wasser-Dampfgemisch in geeigneter Weise zu verwerten.

Kondensiert man das Dampfgemisch und führt das Kondensat in den Reaktor zurück, so nimmt dort der Wasseranteil zu, wobei sich die Konsistenz des Reaktionsgemisches verändert. Anstelle von körnigem Calciumpropionat erhält man ein schlammiges Produkt.

Eine destillative Aufarbeitung des Kondensats ist sehr aufwendig. Hinzu kommt, daß sich dabei eventuelle Verunreinigungen anreichern, die in der Propionsäure enthalten sein können und von dieser destillativ nicht abtrennbar sind.

Die Umsetzung des kondensierten Dampfgemisches mit Calciumhydroxid scheidet ebenfalls aus, da hierbei wegen des hohen Wasseranteils umfangreiche, energieaufwendige Trocknungsprozesse notwendig sind (z. B. Sprühtrocknung).

Es wurde nun gefunden, daß sich ein dampfförmiges Gemisch, das 20 bis 30 Gew.-% Propionsäure und 70 bis 80 Gew.-% Wasser enthält, vorteilhaft zur Herstellung von Calciumpropionat eignet, wenn man dieses Gemisch in eine wäßrige Mischung, die Calciumpropionat, Calciumhydroxid und gegebenenfalls Propionsäure enthält, einleitet, dabei durch weiteren Zusatz von Calciumhydroxid einen pH-Wert zwischen 5 und 10 einstellt und das Calciumpropionat durch Kristallisation isoliert.

Im einzelnen wird das erfindungsgemäße Verfahren beispielsweise wie folgt durchgeführt:

Aus einem kontinuierlich betriebenen Reaktor A, in dem Propionsäure mit Calciumhydroxid umgesetzt wird, entweicht ein Propionsäure-Wasser-Dampfgemisch, dessen Zusammensetzung der des Azeotrops entspricht. Dieses Dampfgemisch enthält in der Regel 10 bis 300 Gew.-% Inertgas, bezogen auf das Propionsäure-Wasser-Dampfgemisch. Als Inertgas wird dabei vorzugsweise Stickstoff verwendet.

Die Zusammensetzung eines azeotropen Gemisches ist in geringem Maße vom Druck abhängig. Im vorliegenden Fall setzt sich dieser aus den Partialdrücken von Propionsäuredampf, Wasserdampf und Inertgas zusammen und ist im Bereich von 0,5 bis 20 bar variabel. Dadurch kann sich auch die Zusammensetzung des Azeotrops zwischen den obengenannten Grenzwerten bewegen.

Mit der im Reaktor A freiwerdenden Reaktionswärme und mittels einer Zusatzheizung wird das dampfförmige Gemisch auf einer Temperatur von 90 bis 165°C erhitzt und in einen Reaktor B geleitet.

Der Reaktor B kann in zwei unterschiedlichen Temperaturbereichen betrieben werden. Eine Unterscheidung in zwei Temperaturbereiche ist angezeigt, weil beim Herstellverfahren zwei verschiedene, temperaturabhängige Aufarbeitungsmethoden möglich sind, die Calciumpropionat in verschiedener Reinheit liefern. Somit richtet sich die Wahl des Temperaturbereichs nach der gewünschten Qualität von Calciumpropionat.

Beim Arbeiten im unteren Temperaturbereich wird das Dampfgemisch mit einem Druck von 0,5 bis 2 bar kontinuierlich in den Reaktor B geleitet. In diesem wird eine wäßrige Mischung gerührt, die 27 bis 50 Gew.-% Calciumpropionat, 0,1 bis 5 Gew.-% Calciumhydroxid und 0,005 bis 1 Gew.-% Propionsäure enthält und deren Temperatur 70 bis 120° C, vorzugsweise 80 bis 100° C beträgt. Dabei liegen vom Calciumpropionat 27 Gew.-% in Lösung und der Rest in Form eines Monohydrats als Feststoff vor. Vom Calciumhydroxid liegen ca. 0,05 Gew.-% in Lösung und der Rest als Feststoff vor. Der pH-Wert der Mischung beträgt 5 bis 10, vorzugsweise 8 bis 9.

Der Reaktor B wird von der wäßrigen Mischung kontinuierlich durchströmt. Ihre Verweilzeit im Reaktor beträgt 0,1 bis 2 Stunden, vorzugsweise 0,2 bis 0,5 Stunden. Aus dem Reaktor entweicht dabei ein Dampfgemisch, dessen Gehalt an Propionsäure $\leq$0,1% ist.

Der Reaktoraustrag, der gelöstes Calciumpropionat und festes Calciumpropionat-monohydrat enthält, wird nach an sich bekannten Methoden aufgetrennt, beispielsweise durch Abfiltrieren, Abdekantieren oder Abschleudern. Anschließend wird der Feststoff nach an sich bekannten Methoden getrocknet und gemahlen, während die Lösung, die mindestens 27 Gew.-% Calciumpropionat enthält, kontinuierlich in den Reaktor B rückgeführt wird.

Der Raum-Zeit-Austrag an Calciumpropionat-monohydrat beträgt dabei 0,1 bis 1 kg pro Stunde, bezogen auf 1 l Reaktorvolumen.

Das bei der Umsetzung verwendete Calciumhydroxid enthält meist Schwermetallsalze, hauptsächlich Eisensalze. Um zu einem schwermetallfreien, hochreinen Endprodukt zu gelangen, muß das Calciumpropionat vollständig gelöst werden, um so die unlöslichen Schwermetallhydroxide abtrennen zu können.

Dies wird vorteilhaft bei einer gesättigten Lösung von Calciumpropionat erreicht. Da aber Calciumpropionat bei 55° C ein Löslichkeitsminimum besitzt, ist es deshalb erforderlich weit über 100° C zu erhitzen und eine gesättigte Lösung bei ca. 200° C zu erzeugen, um somit ein großes Konzentrationsgefälle zu erhalten. Um eine gesättigte Calciumpropionatlösung zu erzeugen, arbeitet man im oberen Temperaturbereich. Dabei wird das Dampfgemisch auf einen Druck von 2 bis 20 bar komprimiert und kontinuierlich in den Reaktor B geleitet, in dem, bei einer Temperatur von 120 bis 210° C, eine wäßrige Mischung gerührt wird, die 27 bis 80 Gew.-% gelöstes Calciumpropionat, 0,1 bis 5 Gew.-% Calciumhydroxid und 0,005 bis 1 Gew.-% Propionsäure enthält. Calciumpropionat ist hierbei vollständig gelöst, während Calciumhydroxid teils in Lösung und teils als Feststoff vorliegt.

Auch hier wird der Reaktor B von der wäßrigen Mischung kontinuierlich durchströmt, wobei die Verweilzeit im Reaktor den oben erwähnten Werten entspricht. Aus dem Reaktor entweicht dabei ein Dampfgemisch, dessen Gehalt an Propionsäure $\leq$0,1% ist.

Der Reaktoraustrag, der gelöstes Calciumpropionat und unlösliche Schwermetallhydroxide enthält, wird bei einer Temperatur von 120 bis 210° C, entsprechend einem Dampfdruck von 2 bis 20 bar filtriert, um so die Verunreinigungen abzutrennen. Dann wird das Filtrat auf 30 bis 80° C abgekühlt, wobei Calciumpropionat-monohydrat ausfällt. Dieses wird wie oben beschrieben abgetrennt und nach an sich bekannten Methoden getrocknet und gemahlen, während die Lösung, die mindestens 27 Gew.-% Calciumpropionat enthält, kontinuierlich in den Reaktor B rückgeführt wird.

Mit dem erfindungsgemäßen neuen Verfahren gelingt es, das bei einem kontinuierlich betriebenen Verfahren zur Herstellung von Calciumpropionat fortwährend anfallende azeotrope Propionsäure-Wasser-Dampfgemisch, ohne Zwischenkondensation direkt in einem ebenfalls kontinuierlich betriebenen Parallelverfahren wiederum zur Herstellung von Calciumpropionat zu verwenden.

Dabei wird in einer Dreiphasenreaktion bei hohem Reaktordurchsatz und sehr niedriger Konzentration sowohl an gelöstem Calciumhydroxid als auch an Propionsäure eine vollständige Umsetzung erzielt.

Die folgenden Beispiele sollen das erfindungsgemäße Verfahren erläutern. Die Prozentangaben in den Beispielen sind Gewichtsprozente.

## Beispiel 1

In einen mit einem Rührer versehenen Absorptionsreaktor aus Edelstahl, dem pro Stunde kontinuierlich 5096 Teile einer wäßrigen Lösung, die 27% Calciumpropionat enthielt, zugeführt wurden, leitete man pro Stunde kontinuierlich 7237,4 Teile eines Dampfgemisches ein, das 25% Propionsäure, 75% Wasser und 126% Stickstoff, bezogen auf das Propionsäure-Wasser-Dampfgemisch, enthielt. Die Temperatur des Dampfgemisches lag bei 138° C, sein Druck bei 1,05 bar.

Zusätzlich wurden über eine Förderschnecke pro Stunde kontinuierlich 400 Teile Calciumhydroxid so zugegeben, daß der pH-Wert der Mischung bei 8 lag.

Die Temperatur des Gemisches betrug 98° C; seine Verweilzeit im Reaktor lag durchschnittlich bei

0,5 Stunden. Aus dem Reaktor entwich dabei ein Dampfgemisch, dessen Gehalt an Propionsäure <0,1% war.

Von der sich im Reaktor befindenden Maische wurden pro Stunde kontinuierlich 6257 Teile ausgetragen und in einer Zentrifuge abgetrennt. Dabei erhielt man pro Stunde 1211 Teile eines rieselfähigen Feststoffs, der 83% Calciumpropionat und 17% Wasser enthielt. Er wurde getrocknet, bis sein Wassergehalt auf ca. 1% gesunken war; anschließend wurde er gemahlen. Als Zentrifugat fielen pro Stunde 5096 Teile einer wäßrigen Lösung an, die 27% Calciumpropionat enthielt. Sie wurde kontinuierlich in den Reaktor rückgeführt.

## Beispiel 2

In einen mit einem Rührer versehenen Absorptionsreaktor aus Edelstahl, dem pro Stunde kontinuierlich 2455 Teile einer Suspension zugeführt wurden, die 400 Teile Calciumhydroxid und 2055 Teile einer 27%igen wäßrigen Calciumpropionatlösung enthielt, leitete man pro Stunde kontinuierlich 7235,9 Teile eines Dampfgemisches ein, das 25% Propionsäure, 75% Wasser und 126% Stickstoff, bezogen auf das Propionsäure-Wasser-Dampfgemisch, enthielt. Die Temperatur des Dampfgemisches lag bei 147° C, sein Druck bei 1,05 bar.

Bevor das Dampfgemisch in den Reaktor eintrat, wurde sein Druck, entsprechend dem Gegendruck des Reaktors, auf 10,2 bar erhöht.

Die Temperatur des Reaktionsgemisches betrug 180° C; seine Verweilzeit im Reaktor lag durchschnittlich bei 0,5 Stunden. Aus dem Reaktor entwich dabei ein Dampfgemisch, dessen Gehalt an Propionsäure <0,1% war.

Von der im Reaktor befindlichen Suspension wurden pro Stunde kontinuierlich 3063 Teile ausgetragen und filtriert, wobei ein Filterrückstand von einem Teil Eisenoxidhydrat anfiel. Das Filtrat wurde auf 55° C abgekühlt, dabei fielen pro Stunde 1005,5 Teile Calciumpropionat als kristalliner Niederschlag aus. Nach dem Entspannen auf Normaldruck wurde in einer Zentrifuge aufgetrennt. Man erhielt pro Stunde 1211 Teile eines hochreinen, rieselfähigen Feststoffs, der 83% Calciumpropionat und 17% Wasser enthielt. Er wurde getrocknet, bis sein Wassergehalt auf ca. 1% gesunken war; anschließend wurde er gemahlen.

Als Zentrifugat fielen pro Stunde 2055 Teile einer wäßrigen Lösung an, die 27% Calciumpropionat enthielt. Sie wurde pro Stunde mit 400 Teilen Calciumhydroxid versetzt und kontinuierlich in den Reaktor rückgeführt.

## Patentansprüche

1. Verfahren zur Herstellung von Calciumpropionat, ausgehend von Calciumhydroxid und Propionsäure, dadurch gekennzeichnet, daß man ein dampfförmiges Gemisch, das 20 bis 30 Gew.-% Propionsäure und 70 bis 80 Gew.-% Wasser enthält, in eine wäßrige Mischung, die Calciumpropionat, Calciumhydroxid und gegebenenfalls Propionsäure enthält, einleitet, dabei durch weiteren Zusatz von Calciumhydroxid einen pH-Wert zwischen 5 und 10 einstellt und das Calciumpropionat durch Kristallisation isoliert.

2. Verfahren gemäß Anspruch 1, dadurch gekennzeichnet, daß man das dampfförmige Gemisch, dessen Temperatur 90 bis 165° C und dessen Druck 0,5 bis 2 bar beträgt, in eine wäßrige, 70 bis 120° C heiße Mischung, die 27 bis 50 Gew.-% Calciumpropionat, 0,1 bis 5 Gew.-% Calciumhydroxid und bis 1 Gew.-% Propionsäure enthält, wobei Calciumpropionat und Calciumhydroxid je teils in Lösung und teils als Feststoffe vorliegen, einleitet, das eingeleitete, sowie das bei der Neutralisation entstandene Wasser dampfförmig abzieht und das Calciumpropionat nach vollständiger Umsetzung des Calciumhydroxids abtrennt und trocknet.

3. Verfahren gemäß Anspruch 1, dadurch gekennzeichnet, daß man das dampfförmige Gemisch, dessen Temperatur 90 bis 165° C beträgt, auf einen Druck von 2 bis 20 bar komprimiert und in eine wäßrige 120 bis 210° C heiße Mischung, einleitet, die 27 bis 80 Gew.-% gelöstes Calciumpropionat, 0,5 bis 5 Gew.-% Calciumhydroxid und bis 1 Gew.-% Propionsäure enthält, wobei Calciumhydroxid teils in Lösung und teils als Feststoff vorliegt, die entstandene Lösung bei einer Temperatur von 120 bis 210° C filtriert, das Filtrat auf eine Temperatur von 30 bis 80° C abkühlt und das ausgefallene Calciumpropionat abtrennt und trocknet.

4. Verfahren gemäß Anspruch 1, dadurch gekennzeichnet, daß man als dampfförmiges Propionsäure-Wasser-Gemisch das Azeotrop verwendet, das in einem kontinuierlich betriebenen Reaktor zur Herstellung von Calciumpropionat aus Calciumhydroxid und Propionsäure verdampft.

## Claims

1. A process for the preparation of calcium propionate starting from calcium hydroxide and propion

ic acid, wherein a vaporous mixture containing from 20 to 30% by weight of propionic acid and from 70 to 80% by weight of water is passed into an aqueous mixture containing calcium propionate, calcium hydroxide and, possibly, propionic acid, during which the pH is brought to 5—10 by further addition of calcium hydroxide, and the calcium propionate is isolated by crystallization.

2. A process as claimed in claim 1, wherein the vaporous mixture, which is at from 90 to 165° C under a pressure of from 0.5 to 2 bar, is passed into an aqueous mixture, at from 70 to 120° C, containing from 27 to 50% by weight of calcium propionate, from 0.1 to 5% by weight of calcium hydroxide and not more than 1% by weight of propionic acid, the calcium propionate and calcium hydroxide each being partly in solution and partly present as solids, the water introduced and that formed during the neutralization is removed as vapor, and, after the calcium hydroxide has been reacted completely, the calcium propionate is separated off and dried.

3. A process as claimed in claim 1, wherein the vaporous mixture, which is at from 90 to 165° C, is compressed to a pressure of from 2 to 20 bar and is passed into an aqueous mixture, at from 120 to 210° C, containing from 27 to 80% by weight of dissolved calcium propionate, from 0.5 to 5% by weight of calcium hydroxide and not more than 1% by weight of propionic acid, the calcium hydroxide being partly in solution and partly present as a solid, the resulting solution is filtered at from 120 to 210° C, the filtrate is cooled to 30—80° C and the calcium propionate which has precipitated is separated off and dried.

4. A process as claimed in claim 1, wherein the azeotrope evaporated off from a continuous-flow reactor for the preparation of calcium propionate from calcium hydroxide and propionic acid is used as the vaporous mixture of propionic acid and water.

**Revendications**

1. Procédé de préparation du propionate de calcium à partir d'acide propionique et d'hydroxyde de calcium, caractérisé en ce que l'on introduit dans un mélange aqueux, contenant du propionate de calcium, de l'hydroxyde de calcium et éventuellement de l'acide propionique, un mélange gazeux contenant entre 20 et 30% en poids d'acide propionique et entre 70 et 80% en poids d'eau, on ajuste le pH de ce mélange entre 5 et 10 par addition d'une proportion supplémentaire d'hydroxyde de calcium, puis on isole le propionate de calcium par cristallisation.

2. Procédé suivant la revendication 1, caractérisé en ce que le mélange gazeux est introduit à une température de 90 à 165° C et sous une pression de 0,5 à 2 bars dans un mélange aqueux, porté à une température comprise entre 70 et 120° C et contenant entre 27 et 50% en poids de propionate de calcium, entre 0,1 et 5% en poids d'hydroxyde de calcium et jusqu'à 1% en poids d'acide propionique, le propionate et l'hydroxyde de calcium étant partiellement dissous et partiellement à l'état solide, l'eau introduite et l'eau formée pendant la neutralisation étant éliminée à l'état gazeux et le propionate de calcium étant séparé et séché après la réaction complète de l'hydroxyde de calcium.

3. Procédé suivant la revendication 1, caractérisé en ce que le mélange gazeux, dont la température se situe entre 90 et 165° C, est comprimé entre 2 et 20 bars, puis introduit dans un mélange aqueux, porté à une température de 120 à 210° C et contenant entre 27 et 80% en poids de propionate de calcium dissous, jusqu'à 1% en poids d'acide propionique et entre 0,5 et 5% en poids d'hydroxyde de calcium, partiellement dissous et partiellement à l'état solide, la solution formée étant filtrée entre 120 et 210° C et le filtrat refroidi entre 30 et 80° C, le propionate de calcium précipité étant séparé et séché.

4. Procédé suivant la revendication 1, caractérisé en ce que l'on emploie en tant que mélange eau-acide propionique gazeux le mélange azéotrope qui se sépare dans un réacteur pour la préparation de propionate de calcium à partir d'acide propionique et d'hydroxyde de calcium, fonctionnant en continu.